# EUROPEAN PATENT APPLICATION

(11) **EP 3 144 368 A1**
(43) Date of publication of application: **22.03.2017**
(21) Application number: 15792946.4
(22) Date of filing: 15.05.2015
(51) Int. Cl.: C10G 2/00, C12N 1/00, C12P 5/02

(54) **LIQUID FUEL PRODUCTION METHOD USING BIOMASS**

(30) Priority: 15.05.2014 JP 2014101840
(71) Applicant: IHI Enviro Corporation, Tokyo 135-0042 (JP); Japan International Research Center for Agricultural Sciences, Tsukuba-shi, Ibaraki 305-8686 (JP)
(72) Inventor: YAMASHITA, Masaharu, Tokyo 135-0042 (JP); KOSUGI, Akihiko, Tsukuba-shi Ibaraki 305-8686 (JP)
(74) Representative: Lamb, Martin John Carstairs
(86) International application number: PCT/JP2015/064021
(87) International publication number: WO 2015/174518

(57) **Abstract**

A liquid fuel production method of the present invention includes: a saccharification step in which a biomass is saccharified; a methane fermentation step in which a saccharified liquid acquired in the saccharification step undergoes methane fermentation; and a biogas to liquid (BTL) step in which a liquid fuel is generated from a biogas acquired in the methane fermentation step.

## Description

### [Technical Field]

The present invention relates to a liquid fuel production method using biomass.

Priority is claimed on Japanese Patent Application No. 2014-101840, filed May 15, 2014, the content of which is incorporated herein by reference.

### [Background Art]

As known in the related art, palm oil is a vegetable oil acquired from the fruit of the oil palm. The main production area of such palm oil is Southeast Asia, where it is produced on large-scale farms known as plantations. For example, Patent Document 1 and Patent Document 2 disclose that bioethanol (a liquid fuel) is produced by performing alcohol fermentation (ethanol fermentation) on sap serving as a raw material acquired from the trunk of an oil palm using the trunk as a biomass (a resource derived from organisms).

### [Document of Related Art]

### [Patent Document]

[Patent Document 1] Japanese Patent No. 4665257
[Patent Document 2] Japanese Patent No. 4418871

### [Summary of Invention]

### [Technical Problem]

However, since the techniques of Patent Document 1 and Patent Document 2 are techniques that focus on alcohol fermentation (ethanol fermentation), energy efficiency is poor in producing bioethanol (a liquid fuel). There is a need to perform a process of concentrating sap (a sugar solution) as a step preceding alcohol fermentation, for example, to improve fermentation efficiency of alcohol fermentation or to prevent spoilage of the sap (the sugar solution) of oil palm, and the concentrating process needs a great deal of energy. Also, a distillation process for separating bioethanol into simple substances is needed as a step subsequent to the alcohol fermentation, and the distillation process also needs a great deal of energy. When a distillation residue separated through a distillation process is used again as feed, or the like, the distillation residue is a solid/liquid mixed solution containing a great amount of water. As such, a great deal of energy is needed to dry the distillation residue.

The present invention was made in view of the above-described circumstances and is for the purpose of improving energy efficiency when a liquid fuel is produced from various biomasses compared to the related art.

### [Solution to Problem]

A first aspect related to a liquid fuel production method using a biomass of the present invention includes: a saccharification step in which the biomass is saccharified; a methane fermentation step in which a saccharified liquid acquired in the saccharification step undergoes methane fermentation; and a biogas to liquid (BTL) step in which a liquid fuel is generated from a biogas acquired in the methane fermentation step.

In a second aspect related to the liquid fuel production method using the biomass of the present invention, in the first aspect, the BTL step includes: a synthesis gas generation step in which a synthesis gas is generated from the biogas acquired in the methane fermentation step; an FT synthesis step in which the synthesis gas acquired in the synthesis gas generation step is subjected to a Fischer-Tropsch (FT) synthesis process; and an upgrade step in which an FT synthetic oil acquired in the FT synthesis step is subjected to an upgrade process.

In a third aspect related to the liquid fuel production method using the biomass of the present invention, in the second aspect, drainage water acquired in the FT synthesis step and the upgrade step is processed in the methane fermentation step.

In a fourth aspect related to the liquid fuel production method using the biomass of the present invention, in any one of the first to third aspects, the liquid fuel production method using the biomass includes: a juicing step of juicing sap from woody biomass including a sugar solution as the sap as a previous step of the saccharification step when the biomass is the woody biomass, wherein, in the methane fermentation step, the saccharified liquid and the sap undergo the methane fermentation.

In a fifth aspect related to the liquid fuel production method using the biomass of the present invention, in any one of the first to fourth aspects, the liquid fuel production method using the biomass further includes: a crushing step of crushing the biomass as the previous step of the saccharification step.

In a sixth aspect related to the liquid fuel production method using the biomass of the present invention, in any one of the first to fifth aspects, the biomass is a trunk of an oil palm and/or a palm oil waste liquid generated from an oil palm in an oil mill for palm oil.

### [Effects of Invention]

According to the present invention, energy efficiency when a liquid fuel is produced from a biomass can be improved compared to the related art. In other words, in the conventional production process in which bioethanol is produced from a biomass such as a palm trunk, a process of concentrating sap (a sugar solution) of an oil palm, a distillation process for separating bioethanol into simple substances, or a drying process of a distillation residue needs a great deal of energy. However, in the present invention, the only step in which an input of external energy is needed is a BTL step. Also, an amount of energy which is needed in the BTL step is significantly smaller than the total amount of energy needed in the concentrating process, the distillation process, and the drying process. Therefore, according to the present invention, since a necessary amount of energy can be reduced compared to the related art in which bioethanol is produced from a biomass such as a palm trunk, total energy efficiency can be improved compared to the related art.

### [Brief Description of Drawings]

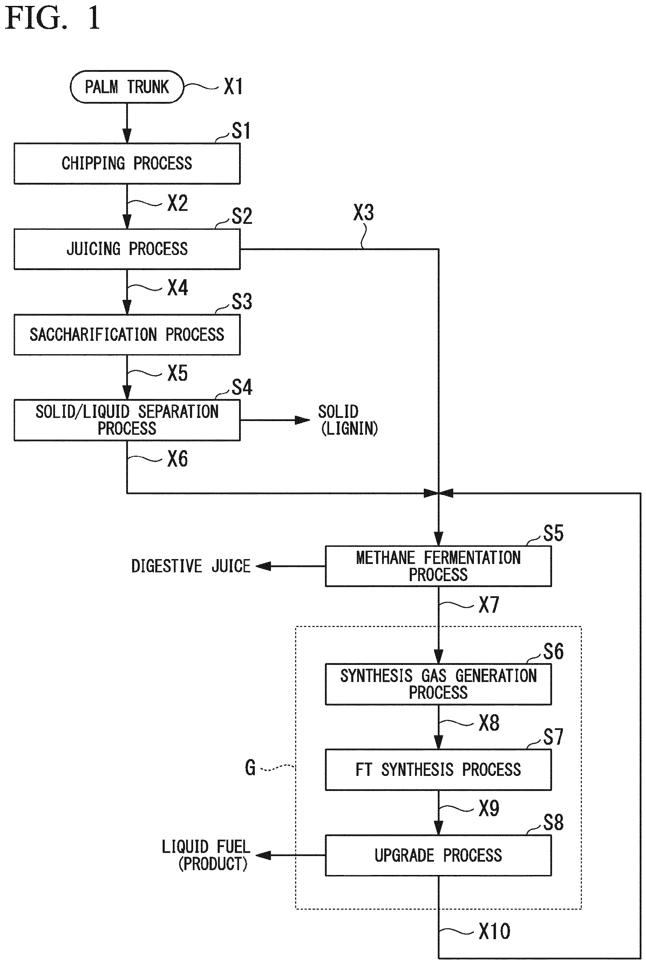

Fig. 1 is a flowchart showing a process of a liquid fuel production method using a biomass related to an embodiment of the present invention.

### [Description of Embodiments]

Hereinafter, an embodiment of the present invention will be described with reference to the accompanying drawings.

As shown in Fig. 1, a liquid fuel production method using a biomass related to the embodiment includes selecting a trunk of oil palm (a palm trunk X1) serving as a type of woody biomass as a biomass (a raw material).

A chipping process S1 serving as a first step is a crushing step in which the log-shaped palm trunk X1 having, for example, a diameter of 30 to 60 cm and a height of about 10 m is crushed into chips (palm chips X2) having, for example, a maximum dimension of about 2.0 to 3.0 mm. The chipping process S1 is mainly performed for the purpose of facilitating a juicing process S2 in the subsequent stage.

In the juicing process S2 serving as a second step, sap X3 is separated from the palm chips X2. In other words, in the juicing process S2, the palm chips X2 are separated into the sap X3 and solids (pulp X4) using a squeezing device. The sap X3 is a sugar solution having water and sugars (the simple sugars pentose and hexose) as main components and is supplied to a methane fermentation process S5 in the subsequent stage as one of raw materials.

In a saccharification process S3 serving as a third step, the pulp X4 acquired in the juicing process S2 is hydrolyzed to be low saccharized (monosaccharification). In the saccharification process S3, the pulp X4 is hydrolyzed on the basis of, for example, an enzyme saccharification method to generate the simple sugars pentose and hexose. As known in the related art, a cellulose-based biomass such as the palm trunk X1 or a woody biomass have cellulose, hemicellulose, and lignin as main components. In an enzyme saccharification method, the cellulose and hemicellulose among the main components are hydrolyzed in the presence of a saccharification enzyme.

In other words, in the saccharification process S3, the cellulose in the pulp X4 is hydrolyzed to generate hexose, while the hemicellulose in the pulp X4 is similarly hydrolyzed to generate pentose. Simple sugars (pentose and hexose) generated by such hydrolysis are soluble in water, and therefore dissolve in water. Therefore, a completely saccharified liquid X5 acquired through the saccharification process S3 is solid/liquid mixed water including a solid having lignin as a main component and a saccharified liquid X6 in which the simple sugars are dissolved into water.

In "a saccharification process" in the embodiment, cellulose or hemicellulose need not necessarily be decomposed into a simple sugar or free sugar, but the pulp X4 (the juice bagasse) may be hydrolyzed to be liquefied or solubilized, and may decompose cellulose or hemicellulose may be decomposed into smaller units than simple sugars or free sugars in the process.

A saccharification process may be a microbial saccharification method using *Clostridium thermocellum*. In particular, the inventors of the present application found that glucan and xylan can be decomposed at 62.5% and 39%, respectively, through a co-culture system of *Clostridium thermocellum* and *Thermoanaerobacter brockii.* For this reason, the saccharification process can be performed through the co-culture system of *Clostridium thermocellum* and *Thermoanaerobacter brockii* with high efficiency.

In a solid/liquid separation process S4 serving as a fourth step, the completely saccharified liquid X5 is separated into solids and liquids. In other words, the saccharified liquid X6 is separated from the completely saccharified liquid X5 using a solid/liquid separation device such a centrifuge. Also, the saccharified liquid X6 is supplied to the methane fermentation process S5 in the subsequent stage as a raw material.

In the methane fermentation process S5 serving as a fifth step, a biogas X7 having methane gas and carbon dioxide as main components is generated through methane fermentation using the sap X3 (the sugar solution) and the saccharified liquid X6 as raw materials. As known in the related art, methane fermentation is a reaction system in which an organic matter is decomposed using an anaerobic organic matter decomposition process, that is, an action of methane bacteria serving as an anaerobic microorganism to generate a digestion gas having methane gas and carbon dioxide as main components.

Here, when components needed for methane fermentation using the sap X3 (the sugar solution) and the saccharified liquid X6 as main components are insufficient, the methane fermentation is not efficiently performed. Thus, the insufficient components in the raw material (for example, nickel, cobalt, molybdenum, etc.) can be appropriately added.

In the methane fermentation process S5, digestive juice is generated as a drainage solution. Components of the digestive juice are determined according to characteristics of raw materials of the methane fermentation process S5, that is, the sap X3 (the sugar solution) and the saccharified liquid X6, but the digestive juice can be processed through an activated sludge process. As known in the related art, an activated sludge process is a technique in which drainage water is processed using an aerobic microorganism and is a waste water treatment method in which digestive juice can be purified up to a river discharge standard. An input of energy from the outside can be suppressed to the minimum using the activated sludge process and the methane fermentation process together.

A synthesis gas generation process S6, an FT synthesis process S7, and an upgrade process S8 which are sixth to eighth steps are included in a biogas to liquid (BTL) step G in which the biogas X7 serving as a gas fuel is converted into a liquid fuel (synthetic naphtha, or the like). In other words, in the synthesis gas generation process S6 serving as the sixth step, a mixed gas (a synthesis gas X8) of hydrogen gas (H₂) and carbon monoxide (CO) is generated from the biogas X7. The biogas X7 is a mixed gas having methane (CH₄) and carbon dioxide (CO₂) as main components, and the synthesis gas X8 can be easily generated using a steam reforming method, a partial oxidation method, etc. which are well known.

In the FT synthesis process S7 serving as the seventh step, the synthesis gas X8 is converted into mixed oil (FT synthetic oil X9) of hydrocarbons having various numbers of carbon atoms. The FT synthetic oil X9 is mixed oil including, for example, methane, ethane, naphtha, kerosene, gas oil, etc. In the FT synthesis process S7, the FT synthetic oil X9 is generated by heating the synthesis gas X8 (a mixed gas of hydrogen gas and carbon monoxide) in the presence of, for example, a metal catalyst, such as iron or cobalt.

In the upgrade process S8 serving as the eighth step, the FT synthetic oil X9 is subjected to a process of distillation into fractions. In the upgrade process S8, for example, the FT synthetic oil is input into a distillation column and heated to be separated into fractions, and the fractions are cooled using water, or the like to be liquefied, and thereby a liquid fuel (a final product) such as synthetic naphtha, synthetic kerosene, and synthetic gas oil is acquired.

In the FT synthesis process S7 and the upgrade process S8, drainage water X10 is generated through a distillation process. The drainage water X10 includes an alcohol or an organic acid as a component and is supplied to the methane fermentation process S5 to be reprocessed (reused) as shown in the drawing. In other words, there is no need to perform a separate drainage process on the drainage water X10 generated in the FT synthesis process S7 and the upgrade process S8.

According to the above-described embodiment, energy efficiency when a liquid fuel serving as a final product is produced from the palm trunk X1 can be improved compared to the related art. In other words, in the conventional production process in which bioethanol is produced from the palm trunk X1, a process of concentrating sap (a sugar solution) of the oil palm, a distillation process for separating bioethanol into simple substances, or a drying process of a distillation residue needs a great deal of energy. However, in the embodiment, the only step in which an input of external energy is needed is the BTL step G. Also, an amount of energy which is needed in the BTL step G is significantly smaller than the total amount of energy needed in the concentrating process, the distillation process, and the drying process. Therefore, according to the embodiment, total energy efficiency can be improved compared to the related art.

Note that the present invention is not limited to the above-described embodiment, and, for example, the following modified examples are considered.
(1) In the liquid fuel production method using the biomass related to the above-described embodiment, the palm trunk X1 serving as a type of woody biomass is used as a raw material, but the present invention is not limited thereto. The present invention can be applied to various biomasses in addition to a woody biomass including a sugar solution as sap.
   For example, cellulose-based biomasses having sap containing sugars include various plants such as crops including palm leaves, bananas, sugar cane, corn, cassava, sago palm, yams, sorghum, potatoes, cellulose and sap (or juice), cellulose/starch/sap (or juice) in addition to the palm trunk X1, and the present invention can be applied to various cellulose-based biomasses.
   Also, the palm trunk X1 is a woody biomass which is discarded from a plantation (a palm plantation) in the course of producing palm oil. The palm trunk X1 and a palm oil waste liquid which can be used as a biomass are generated in the course of producing the palm oil. The palm oil waste liquid is drainage water (palm oil mill effect: POME) having a residue obtained by squeezing crude palm oil (CPO) from the fruit of the oil palm as a main component (sugars, or the like). Therefore, a palm oil waste liquid may be used as a raw material in addition to the palm trunk X1. Thus, a palm oil waste liquid which has been discarded in the related art is used as a raw material of methane fermentation so that environmental impact can be minimized.
(2) In the liquid fuel production method using the biomass related to the above-described embodiment, the synthesis gas generation process S6, the FT synthesis process S7, and the upgrade process S8 are included in the BTL step G for acquiring a liquid fuel (a final product) from the biogas X7, but the present invention is not limited thereto. The biogas X7 may be converted using a method other than the FT method.
(3) In the liquid fuel production method using the biomass related to the above-described embodiment, the chipping process S1 is performed as a first step, but the present invention is not limited thereto. The chipping process S1 can be omitted when, for example, a relatively small solid biomass of chaff or the like or liquid biomass is used as a raw material rather than a relatively large biomass (woody biomass) like the palm trunk X1.
(4) In the liquid fuel production method using the biomass related to the above-described embodiment, the saccharification process S3 and the solid/liquid separation process S4 are performed to acquire the saccharified liquid X6 from the pulp X4, but the present invention is not limited thereto. The saccharification process S3 and the solid/liquid separation process S4 may be omitted, and the methane fermentation process S5 may thus be performed using only the sap X3 as a raw material.
(5) In the BTL step G (the synthesis gas generation process S6, the FT synthesis process S7, and the upgrade process S8) in the above-described embodiment, a gas fuel such as methane or ethane is generated simultaneously with a liquid fuel. Such a gas fuel may be used as a raw material of the synthesis gas generation process S6 together with the biogas X7.

### [Industrial Applicability]

According to the present invention, necessary energy when a liquid fuel is produced from a biomass can be reduced compared to the related art.

### [Description of Reference Signs]

- X1: Palm trunk
- X2: Palm chip
- X3: Sap
- X4: Pulp
- X5: Completely saccharified liquid
- X6: Saccharified liquid
- X7: Biogas
- X8: Synthesis gas
- X9: FT synthetic oil
- X10: Drainage water

## Claims

1. A liquid fuel production method using a biomass, the method comprising:
a saccharification step in which the biomass is saccharified;
a methane fermentation step in which a saccharified liquid acquired in the saccharification step undergoes methane fermentation; and
a biogas to liquid (BTL) step in which a liquid fuel is generated from a biogas acquired in the methane fermentation step.

2. The liquid fuel production method according to claim 1, wherein the BTL step includes:
a synthesis gas generation step in which a synthesis gas is generated from the biogas acquired in the methane fermentation step;
an FT synthesis step in which the synthesis gas acquired in the synthesis gas generation step is subjected to a Fischer-Tropsch (FT) synthesis process; and
an upgrade step in which an FT synthetic oil acquired in the FT synthesis step is subjected to an upgrade process.

3. The liquid fuel production method according to claim 2, wherein drainage water acquired in the FT synthesis step and the upgrade step is processed in the methane fermentation step.

4. The liquid fuel production method according to any one of claims 1 to 3, comprising:
a juicing step of juicing sap from a woody biomass including a sugar solution as the sap as a step preceding the saccharification step when the biomass is the woody biomass,
wherein, in the methane fermentation step, the saccharified liquid and the sap undergo the methane fermentation.

5. The liquid fuel production method according to any one of claims 1 to 4, further comprising:
a crushing step of crushing the biomass as a step preceding the saccharification step.

6. The liquid fuel production method according to any one of claims 1 to 5, wherein the biomass is a trunk of an oil palm and/or a palm oil waste liquid generated from an oil palm in an oil mill for palm oil.
